# EUROPÄISCHE PATENTSCHRIFT

(11) **EP 0 998 884 B1**
(45) Veröffentlichungstag und Bekanntmachung des Hinweises auf die Patenterteilung: **23.06.2004**
(21) Anmeldenummer: 99118498.7
(22) Anmeldetag: 18.09.1999
(51) Int. Cl.: A61F 2/18

(54) **Gehörknöchelprothese**
Auditory ossicles prosthesis
Prothèse d'osselets de l'oreille

(30) Priorität: 06.11.1998 DE 29819892 U
(43) Veröffentlichungstag der Anmeldung: 10.05.2000
(73) Patentinhaber: Heinz Kurz GmbH Medizintechnik, 72144 Dusslingen (DE)
(72) Erfinder: Kurz, Heinz, 72144 Dusslingen (DE); Gamer, Walter, 76646 Bruchsal (DE)
(74) Vertreter: Möbus, Daniela, Dr.-Ing.

(56) Entgegenhaltungen:
- EP-A- 0 379 740
- EP-A- 0 460 354
- EP-A- 0 563 767
- WO-A-92/18066
- DE-A- 19 744 789
- DE-U- 29 802 776
- FR-A- 2 721 199
- US-A- 5 554 188
- US-A- 5 814 104

## Beschreibung

Die Erfindung betrifft eine Gehörknöchelprothese mit einer zur Anlage am Trommelfell ausgebildeten Kopfplatte und einem darauf angeordneten Schaft zur Überbrückung der Paukenhöhle vom Trommelfell bis zum Steigbügel oder bis zur Steigbügelfußplatte, wobei der Schaft an seinem steigbügelseitigen Ende ein Kopplungsstück aufweist.

Eine Mittelohr-Komplettprothese einschließlich Trommelfell-Ersatz ist bekannt aus der EP-A-0 460 354.

Bei der Implantation von Gehörknöchelprothesen muss vor der Implantation die Länge der Prothese individuell auf den jeweiligen Patienten abgestimmt werden. Da man die erforderliche Länge aber erst während der Operation bestimmen kann, muss für jede Operation entweder ein Satz von Prothesen unterschiedlicher Länge oder eine Prothese mit variabler Schaftlänge bereitgestellt werden. Bei einer bisher bekannten, längenveränderlichen Gehörknöchelprothese ist der Schaft der Prothese in mehrere aufeinander folgende Abschnitte unterteilt, wobei die aufeinander folgenden Abschnitte mittels einer Querschnittsverjüngung als Sollbruchstellen voneinander getrennt sind. Weil bei den filigranen Gehörknöchelprothesen jedoch im Bereich von Zehntel und Hundertstel Millimetern gearbeitet wird, ist die Fertigung der Sollbruchstellen verhältnismäßig aufwendig und damit teuer.

Die Erfindung hat die Aufgabe, eine Gehörknöchelprothese der eingangs genannten Art dahin gehend zu verbessern, dass das Ablängen des Schaftes auf die individuelle Länge mit einem geringen Fertigungsaufwand für den Schaft und damit kostengünstig erfolgen kann.

Die Erfindung löst die gestellte Aufgabe mit einer Gehörknöchelprothese der eingangs genannten Art, bei der die Kopfplatte eine Durchgangsbohrung zur Aufnahme des Schaftes aufweist, durch die der längliche Schaft hindurchgeschoben werden kann, sodass er auf der Außenseite der Kopfplatte übersteht und dort abgelängt werden kann, wobei die Bohrung verengbar ist, um den Schaft zu fixieren. Anschließend nach der Verkürzung des Schaftes wird die Bohrung der Kopfplatte verengt und dadurch der Schaft sicher fixiert. Danach kann die angepasste Prothese dem Patienten implantiert werden.

Die erfindungsgemäße Gehörknöchelprothese ist somit rasch und stufenlos an die anatomischen Gegebenheiten des Patienten anpassbar.

Zur Verengung der Bohrung in der Kopfplatte gibt es verschiedene Möglichkeiten. So kann der Umfang der Bohrung durch einen Schlitz unterbrochen und der Schlitz und damit die Bohrung über einen verformbaren Steg durch Verstemmen des Steges verengbar sein. Dadurch kann der Schaft mit wenig Aufwand rasch an der Kopfplatte fixiert werden. Aus demselben Grund kann die Bohrung in der Mitte eines Schlitzes liegen und die Bohrung durch Zusammendrücken des Schlitzes verengbar sein. Die Bohrung kann auch in der Mitte von zwei sich kreuzenden Schlitzen liegen, wobei die Bohrung einen kleineren Durchmesser als der Schaftdurchmesser aufweist.

Damit die Kopfplatte optimal auf dem Trommelfell aufliegen kann, kann sie elliptisch sein und abgerundete Kanten haben sowie mindestens einen Durchbruch aufweisen, damit der Operateur die Position des Kopplungsstückes kontrollieren kann.

Zur Erleichterung der Einstellung der Länge des Schaftes kann der Schaft Skalierungen aufweisen.

Damit die Gehörknöchelprothese optimal am Trommelfell oder am Steigbügel anliegt, kann sie an der dem Trommelfell und/oder Steigbügel zugewandten Seite angeraut sein.

Aus Gründen einer kostengünstigen Fertigung kann die Gehörknöchelprothese lasertechnisch hergestellt werden.

Damit die Gehörknöchelprothese körperverträglich ist, kann sie aus Gold und/oder Titan hergestellt sein.

Das Kopplungsstück der Gehörknöchelprothese kann in Form eines glockenförmigen Gebildes oder eines Stempels ausgebildet sein. Somit kann die Gehörknöchelprothese sowohl als Total- als auch als Partialprothese zuverlässig am Steigbügel befestigt werden.

Bei Verwendung eines glockenförmigen Kopplungsstückes kann dieses zur Gewährleistung eines optimalen Halts am Steigbügel geschlitzt sein, wobei der Schlitz parallel zur längeren Halbachse der elliptischen Kopfplatte verlaufen und der Schlitz in dem glockenförmigen Gebilde vorzugsweise 0,6 mm breit sein kann.

Bei Ausbildung der Prothese als Totalprothese mit einem stempelförmigen Kopplungsstück kann dieses einen Durchmesser von 0,8 mm und einen Hohlraum aufweisen, um einen guten Sitz an der Steigbügelfußplatte zu erzielen.

Zur Einstellung der Schaftlänge dieser Gehörknöchelprothese schlägt die Erfindung ein Werkzeug vor, das zwei gelenkig miteinander verbundene Schenkel aufweist, wobei sich die Dicke der Schenkel stufenförmig über die Schenkellänge verändert und auf der Innenseite der Schenkel Vertiefungen zur Aufnahme des Schaftes und seitlich Vertiefungen zur Aufnahme der Kopfplatte angeordnet sind. Somit kann also der Schaft der Gehorknochelprothese in eine der Vertiefungen zur Aufnahme des Schaftes eingelegt werden, und zwar an einer der Stufen der Schenkel des Werkzeuges, deren Dicke genau der gewünschten Länge des Schaftes entspricht. Auf diese Weise hat also das Werkzeug zur Einstellung der Schaftlänge die Funktion einer Lehre. Durch dieses spezielle Werkzeug und den individuell ablängbaren Schaft kann die Schaftlänge der Gehörknöchelprothese während der Operation in kürzester Zeit an die genauen Abmessungen im Ohr des Patienten angepasst werden.

Damit der Schaft optimal in die Vertiefungen an der Kontaktfläche zum anderen Schenkel hineinpasst, können die Vertiefungen an der Kontaktfläche zum anderen Schenkel als halbkreisförmige Ausnehmungen ausgebildet sein.

Nachfolgend wird ein Ausführungsbeispiel einer erfindungsgemäß ausgebildeten Gehörknöchelprothese und eines Werkzeuges anhand der beiliegenden Zeichnung näher beschrieben.

Im Einzelnen zeigen:
- Fig. 1: eine Seitenansicht auf eine Gehörknöchelprothese;
- Fig. 2: eine Detailansicht auf ein Kopplungsstück;
- Fig. 3: eine Draufsicht auf eine Kopfplatte;
- Fig. 4: eine Draufsicht auf eine zweite Ausführungsform einer Kopfplatte;
- Fig. 5: eine Draufsicht auf eine dritte Ausführungsform einer Kopfplatte;
- Fig. 6: eine Draufsicht auf ein Werkzeug zur Einstellung der Schaftlänge einer Gehörknöchelprothese;
- Fig. 7: eine Seitenansicht auf einen Schenkel des Werkzeuges aus Fig. 6;
- Fig. 8: eine Ansicht von unten auf das Werkzeug aus Fig. 6.

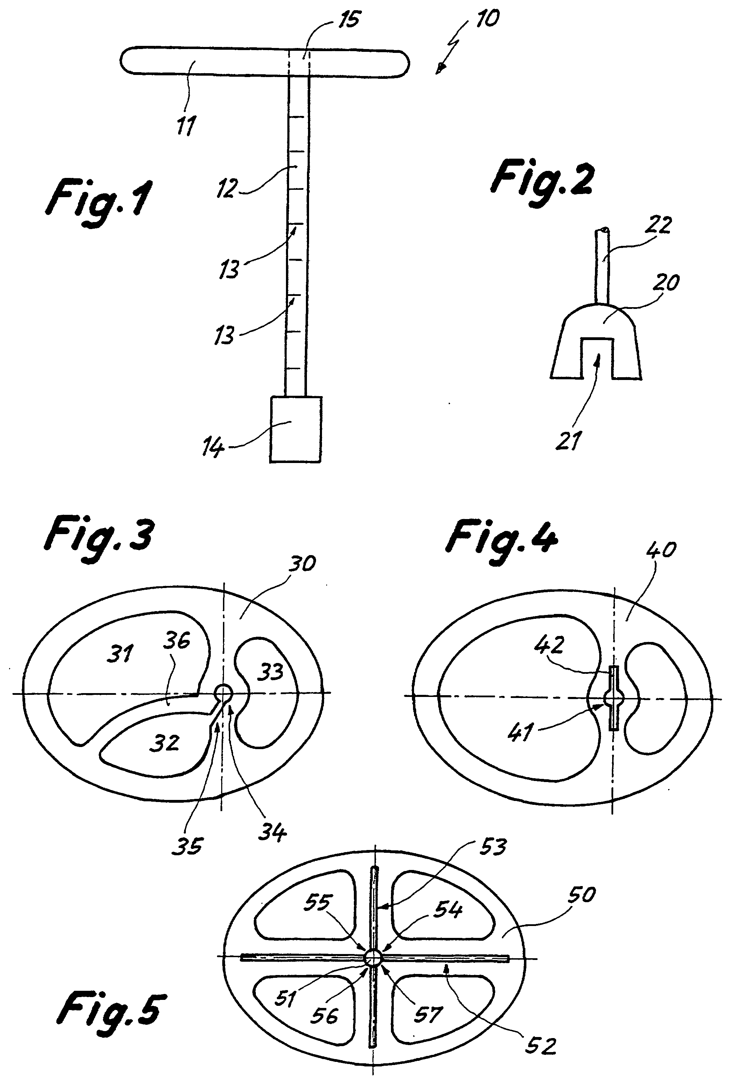

Fig. 1 zeigt eine Gehörknöchelprothese 10 mit einer Kopfplatte 11, die an das Trommelfell angelegt wird. An der Kopfplatte 11 ist ein Schaft 12 in einer Bohrung 15 angeordnet, der die Paukenhöhle vom Trommelfell bis zum Steigbügel oder bis zur Steigbügelfußplatte überbrückt. Der Schaft 12 weist Skalierungen 13 auf, mittels denen die gewünschte Schaftlänge bestimmt werden kann. Am unteren Ende des Schaftes 12 ist für den Einsatz als Totalprothese ein Stempel 14 angebracht. Der Stempel 14 wird bei der Implantation an der Steigbügelfußplatte angeordnet.

Fig. 2 zeigt eine Detailansicht eines glockenförmig ausgebildeten Kopplungsstückes 20. Das Kopplungsstück 20 wird bei Einsatz der Prothese als Partialprothese verwendet und am Steigbügel angeordnet. Aus Gründen eines optimalen Sitzes des Kopplungsstückes 20 an dem Steigbügel weist das Kopplungsstück 20 einen Schlitz 21 auf. Das Kopplungsstück 20 ist an einen Schaft 22 montiert.

Fig. 3 zeigt eine Kopfplatte 30, die am Trommelfell angelegt wird. Die Kopfplatte 30 weist eine ovale Form auf und ist an der dem Trommelfell zugewandten Seite angeraut, damit sie optimal auf dem Trommelfell aufliegen kann. Ferner weist die Kopfplatte 30 Durchbrüche 31, 32 und 33 auf. Durch eine Durchgangsbohrung 34 kann der Schaft 12, 22 einer Gehörknöchelprothese 10 hindurchgeführt werden. Der Umfang der Durchgangsbohrung 34 ist durch einen Schlitz 35 unterbrochen. Mittels eines verformbaren Steges 36 kann der Schlitz und damit die Bohrung 34 durch Verstemmen des Steges 36 verengt werden. Auf diese Weise wird der in der Durchgangsbohrung 34 eingeführte Schaft fixiert. Solange die Bohrung 34 noch nicht verengt worden ist, lässt sich der Schaft 12, 22 jedoch durch die Bohrung 34 hindurchschieben, sodass sein Ende auf der Außenseite der Kopfplatte 30 übersteht und abgeschnitten werden kann.

Fig. 4 zeigt eine andere Ausgestaltung einer Kopfplatte 40, die eine Bohrung 41 in der Mitte eines Schlitzes 42 aufweist. Durch Zusammendrücken des Schlitzes 42 und der Bohrung 41 kann der durch die Bohrung 41 eingeführte Schaft fixiert werden.

Fig. 5 zeigt eine Kopfplatte 50, die eine Bohrung 51 in der Mitte zweier sich kreuzender Schlitze 52 und 53 aufweist. Durch die Bohrung 51 wird ein Schaft einer Gehörknöchelprothese hindurchgeführt, dessen Durchmesser größer ist als der Durchmesser der Bohrung 51. Somit kann sich der Schaft entgegen der Durchführungsrichtung an den abgerundeten Ecken 54, 55, 56 und 57 verkeilen, womit er entgegen der Durchführungsrichtung fixiert wird.

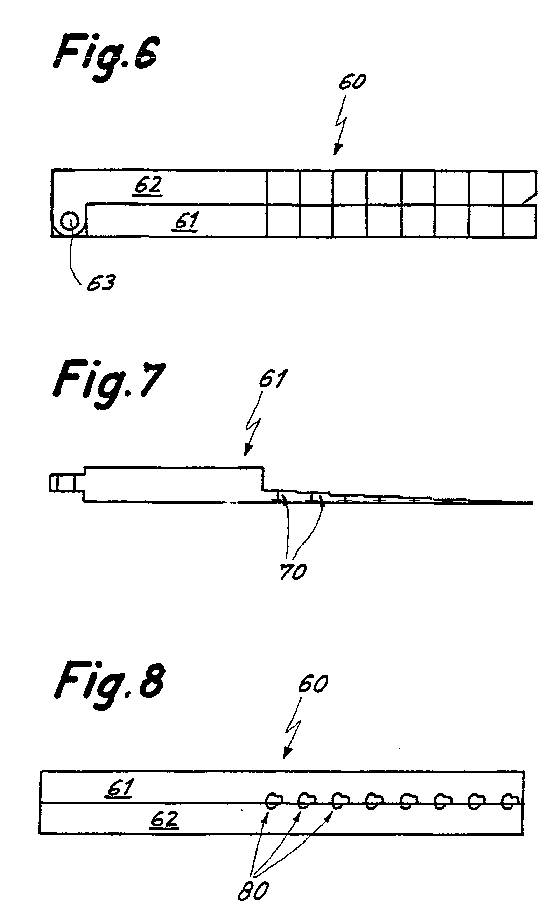

Fig. 6 zeigt ein Werkzeug 60 zur Einstellung der Schaftlänge einer Gehörknöchelprothese. Das Werkzeug 60 weist zwei Schenkel 61 und 62 auf, die über ein Gelenk 63 miteinander verbunden sind.

Fig. 7 zeigt den Schenkel 61, dessen Dicke sich genauso wie beim hier nicht dargestellten Schenkel 62 stufenförmig über die Schenkellänge verändert. Die Dicke der einzelnen Stufen entspricht der gewünschten Schaftlänge der Gehörknöchelprothese. An dem Schenkel 61 wie auch an dem hier nicht näher dargestellten Schenkel 62 sind Vertiefungen 70 angebracht. Diese Vertiefungen sind vorzugsweise als halbkreisförmige Ausnehmungen ausgebildet. In diese halbkreisförmig ausgebildeten Ausnehmungen kann beim Ablängen des Prothesenschaftes der Schaft eingelegt werden, bevor die beiden Schenkel geschlossen werden. Der überstehende Bereich des Schaftes wird dann mittels eines Skalpells oder einer Schere abgeschnitten.

Fig. 8 zeigt das Werkzeug 60 zur Einstellung der Schaftlänge von unten. Die beiden Schenkel 61 und 62 sind geschlossen. Das Werkzeug 60 weist Vertiefungen 80 zur Aufnahme der Kopfplatte auf. In den Vertiefungen 80 wird die Kopfplatte der Gehörknöchelprothese eingespannt, damit sie mit dem an ihr befestigten Schaft nicht aus dem Werkzeug fallen kann.

## Patentansprüche

1. Gehörknöchelprothese (10) mit einer zur Anlage am Trommelfell ausgebildeten Kopfplatte (30, 40, 50) und einem darauf angeordneten Schaft (12, 22) zur Überbrückung der Paukenhöhle vom Trommelfell bis zum Steigbügel oder bis zur Steigbügelfußplatte, wobei der Schaft (12, 22) an seinem steigbügelseitigen Ende ein Kopplungsstück (14, 20) aufweist, **dadurch gekennzeichnet, dass** die Kopfplatte (30, 40, 50) eine Durchgangsbohrung (15, 34, 41, 51) zur Aufnahme des Schaftes (12, 22) aufweist, durch die der längliche Schaft (12, 22) hindurchgeschoben werden kann, sodass er auf der Außenseite der Kopfplatte (30, 40, 50) übersteht und dort abgelängt werden kann, wobei die Bohrung (15, 34, 41, 51) verengbar ist, um den Schaft (12, 22) zu fixieren.

2. Gehörknöchelprothese (10) nach Anspruch 1, **dadurch gekennzeichnet, dass** der Umfang der Bohrung (34) durch einen Schlitz (35) unterbrochen ist und der Schlitz (35) und damit die Bohrung (34) über einen verformbaren Steg (36) durch Verstemmen des Steges (36) verengbar ist.

3. Gehörknöchelprothese (10) nach Anspruch 1, **dadurch gekennzeichnet, dass** die Bohrung (41) in der Mitte eines Schlitzes (42) liegt und die Bohrung (41) durch Zusammendrücken des Schlitzes (42) verengbar ist.

4. Gehörknöchelprothese (10) nach Anspruch 1, **dadurch gekennzeichnet, dass** die Bohrung (51) in der Mitte von zwei sich kreuzenden Schlitzen (52, 53) liegt, wobei die Bohrung (51) einen kleineren Durchmesser als der Schaftdurchmesser aufweist.

5. Gehörknöchelprothese (10) nach einem der Ansprüche 1 bis 4, **dadurch gekennzeichnet, dass** die Kopfplatte (30, 40, 50) elliptisch ist und abgerundete Kanten hat.

6. Gehörknöchelprothese (10) nach einem der Ansprüche 1 bis 5, **dadurch gekennzeichnet, dass** die Kopfplatte (30, 40, 50) mindestens einen Durchbruch aufweist.

7. Gehörknöchelprothese (10) nach einem der Ansprüche 1 bis 6, **dadurch gekennzeichnet, dass** der Schaft (12, 22) Skalierungen (13) aufweist.

8. Gehörknöchelprothese (10) nach einem der Ansprüche 1 bis 7, **dadurch gekennzeichnet, dass** sie an der dem Trommelfell und/oder Steigbügel zugewandten Seite angeraut ist.

9. Gehörknöchelprothese (10) nach einem der Ansprüche 1 bis 8, **dadurch gekennzeichnet, dass** sie lasertechnisch herstellbar ist.

10. Gehörknöchelprothese (10) nach einem der Ansprüche 1 bis 9, **dadurch gekennzeichnet, dass** sie aus Gold und/oder Titan hergestellt ist.

11. Gehörknöchelprothese (10) nach einem der Ansprüche 1 bis 10, **dadurch gekennzeichnet, dass** das Kopplungsstück (14, 20) in Form eines glockenförmigen Gebildes (20) oder eines Stempels (14) ausgebildet ist.

12. Gehörknöchelprothese (10) nach Anspruch 11, **dadurch gekennzeichnet, dass** das glockenförmige Gebilde (20) geschlitzt ist, wobei der Schlitz (21) parallel zur längeren Halbachse der elliptischen Kopfplatte (30, 40, 50) verläuft.

13. Gehörknöchelprothese (10) nach Anspruch 11 oder 12, **dadurch gekennzeichnet, dass** der Schlitz (21) in dem glockenförmigen Gebilde (20) 0,6 mm breit ist.

14. Gehörknöchelprothese (10) nach einem der Ansprüche 1 bis 11, **dadurch gekennzeichnet, dass** der Stempel (14) einen Durchmesser von 0,8 mm und einen Hohlraum aufweist.

15. Gehörknöchelprothese (10) nach einem der Ansprüche 1 bis 14, mit einem Werkzeug (60) zur Einstellung der Schaftlänge, das zwei gelenkig miteinander verbundene Schenkel (61, 62) aufweist, wobei sich die Dicke der Schenkel (61, 62) stufenförmig über die Schenkellänge verändert und auf der Innenseite der Schenkel (61, 62) Vertiefungen (70) zur Aufnahme des Schaftes (12, 22) und seitlich zur Aufnahme der Kopfplatte (30, 40, 50) angeordnet sind.

16. Gehörknöchelprothese (10) mit Werkzeug (60) nach Anspruch 15, **dadurch gekennzeichnet, dass** die Vertiefungen (70) an der Kontaktfläche zum anderen Schenkel (61, 62) als halbkreisförmige Ausnehmungen ausgebildet sind.

## Claims

1. Auditory ossicles prosthesis (10) having a head plate (30, 40, 50) formed to abut against the ear drum and a shaft (12, 22) arranged on the head plate (30, 40, 50) for bridging the tympanic cavity from the ear drum up to the stirrup or up to the stirrup foot plate, the shaft (12, 22) having a coupling member (14, 20) at its end towards the stirrup, **characterised in that** the head plate (30, 40, 50) has a through hole (15, 34, 41, 51) for receiving the shaft (12, 22) through which the elongate shaft (12, 22) can be displaced so that it projects beyond the outer surface of the head plate (30, 40, 50) and can there be cut to length, the hole (15, 34, 41, 51) being narrowable for fixing the shaft (12, 22) in position.

2. Auditory ossicles prosthesis (10) according to claim 1, **characterised in that** the periphery of the hole (34) is interrupted by a slot (35), and the slot (35) and with it the hole (34) can be narrowed by way of a deformable web (36) by spreading the web (36).

3. Auditory ossicles prosthesis (10) according to claim 1, **characterised in that** the hole (41) is disposed in the middle of a slot (42) and the hole (41) can be narrowed by pressing the slot (42) together.

4. Auditory ossicles prosthesis (10) according to claim 1, **characterised in that** the hole (51) is disposed in the middle of two intersecting slots (52, 53), the hole (51) being of smaller diameter than the shaft diameter.

5. Auditory ossicles prosthesis (10) according to one of claims 1 to 4, **characterised in that** the head plate (30, 40, 50) is elliptical and has rounded edges.

6. Auditory ossicles prosthesis (10) according to one of claims 1 to 5, **characterised in that** the head plate (30, 40, 50) has at least one opening.

7. Auditory ossicles prosthesis (10) according to one of claims 1 to 6, **characterised in that** the shaft (12, 22) is provided with a scale (13).

8. Auditory ossicles prosthesis (10) according to one of claims 1 to 7, **characterised in that** it is roughened at the side facing the ear drum and/or the stirrup.

9. Auditory ossicles prosthesis (10) according to one of claims 1 to 8, **characterised in that** it can be produced by laser technology.

10. Auditory ossicles prosthesis (10) according to one of claims 1 to 9, **characterised in that** it is made of gold and/or titanium.

11. Auditory ossicles prosthesis (10) according to one of claims 1 to 10, **characterised in that** the coupling member (14, 20) is made as a bell shaped element (20) or shaped as a plunger (14).

12. Auditory ossicles prosthesis (10) according to claim 11,
**characterised in that** the bell shaped element (20) is slotted, the slot (21) extending parallel to the longer semi axis of the elliptical head plate (30, 40, 50).

13. Auditory ossicles prosthesis (10) according to claim 11 or 12, **characterised in that** the slot (21) in the bell shaped element (20) is 0.6 mm in width.

14. Auditory ossicles prosthesis (10) according to one of claims 1 to 11, **characterised in that** the plunger (14) is 0.8 mm in diameter and has a cavity.

15. Auditory ossicles prosthesis (10) according to one of claims 1 to 14, with a tool (60) for adjusting the shaft length, the tool (60) having two articulatedly interconnected limbs (61, 62), the thickness of the limbs (61, 62) changing in steps along the length of the limbs, depressions (70) being arranged on the inner surface of the limbs (61, 62) for receiving the shaft (12, 22) and laterally for receiving the head plate (30, 40, 50).

16. Auditory ossicles prosthesis (10) with tool (60) according to claim 15, **characterised in that** the depressions (70) at the contact surface facing the other limb (61, 62) are formed as semi circular recesses.

## Revendications

1. Prothèse d'osselets auditifs (10) avec une plaque de tête (30, 40, 50) conçue pour être appliquée sur la membrane tympanique et avec une tige (12, 22) fixée dessus pour recouvrir la caisse tympanique de la membrane tympanique à l'étrier ou à la plaque de pied de l'étrier, la tige (12, 22) comportant à son extrémité côté étrier une pièce d'accouplement (14, 20), **caractérisée en ce que**, pour recevoir la tige (12, 22), la plaque de tête (30, 40, 50) comporte un trou débouchant (15, 34, 41, 51) à travers lequel la tige allongée (12, 22) peut être enfilée de façon qu'elle dépasse sur le côté extérieur de la plaque de tête (30, 40, 50) et puisse y être mise à longueur, le trou (15, 34, 41, 51) étant rétrécissable pour immobiliser la tige (12, 22).

2. Prothèse d'osselets auditifs (10) selon la revendication 1, **caractérisée en ce que** la circonférence du trou (34) est interrompue par une fente (35) et la fente (35) et donc le trou (34) sont rétrécissables par l'intermédiaire d'un pont déformable (36) par extension du pont (36).

3. Prothèse d'osselets auditifs (10) selon la revendication 1, **caractérisée en ce que** le trou (41) se trouve au centre d'une fente (42) et le trou (41) est rétrécissable par compression de la fente (42).

4. Prothèse d'osselets auditifs (10) selon la revendication 1, **caractérisée en ce que** le trou (51) se trouve au centre de deux fentes croisées (52, 53), le trou (51) présentant un diamètre inférieur au diamètre de la tige.

5. Prothèse d'osselets auditifs (10) selon l'une des revendications 1 à 4, **caractérisée en ce que** la plaque de tête (30, 40, 50) est elliptique et a des bords arrondis.

6. Prothèse d'osselets auditifs (10) selon l'une des revendications 1 à 5, **caractérisée en ce que** la plaque de tête (30, 40, 50) comporte au moins un passage.

7. Prothèse d'osselets auditifs (10) selon l'une des revendications 1 à 6, **caractérisée en ce que** la tige (12, 22) comporte des graduations (13).

8. Prothèse d'osselets auditifs (10) selon l'une des revendications 1 à 7, **caractérisée en ce qu'**elle est rendue rugueuse sur le côté tourné vers la membrane tympanique et/ou l'étrier.

9. Prothèse d'osselets auditifs (10) selon l'une des revendications 1 à 8, **caractérisée en ce qu'**elle est réalisable par technique au laser.

10. Prothèse d'osselets auditifs (10) selon l'une des revendications 1 à 9, **caractérisée en ce qu'**elle est réalisée en or et/ou en titane.

11. Prothèse d'osselets auditifs (10) selon l'une des revendications 1 à 10, **caractérisée en ce que** la pièce d'accouplement (14, 20) est conformée en structure en forme de cloche (20) ou en poinçon (14).

12. Prothèse d'osselets auditifs (10) selon la revendication 11, **caractérisée en ce que** la structure en forme de cloche (20) est fendue, la fente (21) s'étendant parallèlement au plus long demi-axe de la plaque de tête elliptique (30, 40, 50).

13. Prothèse d'osselets auditifs (10) selon la revendication 11 ou 12, **caractérisée en ce que** la fente (21) dans la structure en forme de cloche (20) est large de 0,6 mm.

14. Prothèse d'osselets auditifs (10) selon l'une des revendications 1 à 11, **caractérisée en ce que** le poinçon (14) présente un diamètre de 0,8 mm et une cavité.

15. Prothèse d'osselets auditifs (10) selon l'une des revendications 1 à 14, avec un outil (60) pour le réglage de la longueur de tige, lequel comporte deux branches (61, 62) reliées entre elles de manière articulée, l'épaisseur des branches (61, 62) variant par paliers sur la longueur des branches et des renfoncements (70) étant disposés sur le côté intérieur des branches (61, 62) pour la réception de la tige (12, 22) et latéralement pour la réception de la plaque de tête (30, 40, 50).

16. Prothèse d'osselets auditifs (10) avec outil (60) selon la revendication 15, **caractérisée en ce que** les renfoncements (70) sur la surface de contact avec l'autre branche (61, 62) sont conformés en évidements semi-circulaires.
